# EUROPEAN PATENT APPLICATION

(11) **EP 3 928 778 A1**
(43) Date of publication of application: **29.12.2021**
(21) Application number: 20764917.9
(22) Date of filing: 08.06.2020
(51) Int. Cl.: A61K 31/4965, A61K 9/20, A61K 9/48, A61K 47/38, A61P 31/14

(54) **ANTI-RNA VIRAL PHARMACEUTICAL COMPOSITION AVIFAVIR EFFECTIVE, INTER ALIA, AGAINST SARS-COV-2**

(30) Priority: 07.05.2020 RU 2020116521
(71) Applicant: Cromis, Limited Liability Company, Moscow 121205 (RU)
(72) Inventor: IVASHCHENKO, Andrey Alexandrovich, Moscow 127576 (RU); SAVCHUK, Nikolay Filippovich, Rancho Santa Fe California 92067 (US); IVACHTCHENKO, Alena Alexandrovna, Hallandale Florida 33009 (US); IVACHTCHENKO, Alexandre Vasilievich, Hallandale Florida 33009 (US); IL'IN, Aleksei Petrovich, Moskovskaya obl. Khimki 125565 (RU); KRAVCHENKO, Dmitrii Vladimirovich, Moskovskaya obl. g. Khimki 141402 (RU); PAPAZOVA, Natalia Aleksandrovna, Moskovskaya obl. g. Liubertsy 140000 (RU); SITDEKOV, Tagir Alievich, Moscow 109147 (RU)
(74) Representative: Zellentin & Partner mbB Patentanwälte
(86) International application number: PCT/RU2020/000270
(87) International publication number: WO 2021/225463

(57) **Abstract**

This invention relates to a novel anti-RNA virus, including anti-SARS-CoV-2 virus, pharmaceutical composition Avifavir in tablets or capsules containing less than 50 wt % of micronized favipiravir, with the remainder being excipients.

Medicinal product Avifavir for the prevention and treatment of COVID-19 coronaviral disease, said medicinal product being a pharmaceutical composition in coated tablets containing 200 mg, 300 mg, 400 mg, or 600 mg of micronized favipiravir (less than 40-45%) with a particle size of less than 60 µm, with the remainder being excipients.

## Description

This invention relates to a novel anti-RNA virus, including anti-SARS-CoV-2 virus, pharmaceutical composition Avifavir intended to treat RNA viral diseases, including to prevent and treat COVID-19 and highly pathogenic influenza viruses. A sudden outbreak of the new coronavirus (later named SARS-CoV-2) in Wuhan, China, in 2019, which quickly turned into a global pandemic, marked the third introduction of a virulent coronavirus into human society, affecting not only the health system, but also the global economy. Effective approaches to vaccination, prevention, and treatment of SARS-CoV-2 (COVID-19) and epidemiological control are still lacking.

In this regard, an intensive search for vaccines and therapeutic agents for the prevention and treatment of SARS-CoV-2 (COVID-19) is underway worldwide. One practical approach as a rapid response to an emerging pandemic is to repurpose existing therapeutic agents previously intended for other viral infections, since most of these agents have already been tested for their safety.

A well-known anti-influenza drug is Favipiravir (FRP, T-705, Avigan) patented in 1999 by the Japanese company Toyama Chemical Co [RU 2224520]. FVP is used in Japan to treat influenza, including the highly pathogenic A H5N1 strain of avian influenza [R.W. Sidwell et al. Antimicrob. Agents Chemother. 2007, 51(3): 845-851].

FVP shows antiviral activity against many other RNA viruses, such as arenaviruses, bunyaviruses and filoviruses, which are known to cause fatal hemorrhagic fever [Y. Furuta et al. Review Favipiravir (T-705), a broad spectrum inhibitor of viral RNA polymerase. Proc. Jpn. Acad., Ser.B 93, 2017, 449-463].

FVP has successfully passed a trial to treat a progressive infection in mice caused by the Ebola virus [L. Oestereich et al. Successful Treatment of Advanced Ebola Virus Infection with T-705 (Favipiravir) in a Small Animal Model. Antiviral Research 2014, 105, 17-21.] and is an encouraging drug candidate, but has not yet been approved by WHO.

Ebola Virus Disease (EVD), also known as Ebola Hemorrhagic Fever (EHF) or just Ebola fever, is a viral hemorrhagic fever of humans and other primates caused by ebolaviruses. The disease has a high risk of death, killing 25% to 90% of infected individuals, with an average of about 50%. EVD outbreaks occur intermittently in tropical regions of sub-Saharan Africa. Between 1976 and 2013, WHO reports 24 outbreaks involving 2,387 cases with 1,590 deaths. The largest outbreak to date was the epidemic in West Africa, which occurred from December 2013 to January 2016, with 28,646 cases and 11,323 deaths. An Ebola vaccine was approved in the United States in December 2019, while there is no approved treatment for Ebola as of 2019 [https://en.wikipedia.org/wiki/Ebola_virus_disease].

In February 2020, FVP was successfully tested in China as an antiviral therapy against SARS-CoV-2 (COVID-19) in a first non-randomized study. On February 16, 2020, FVP got a short-term approval in China as a potent antiviral drug against COVID-19 to be used for five years. It is now produced in China as Favilavir [https://de.wikipedia.org/wiki/Favipiravir].

FVP, sold in Japan under the brand name Avigan (Avigan Tablet 200 mg, Toyama Chemical Co.) and in China under the brand name Favilavir (Favilavir Tablet 200 mg, Zhejiang Hisun Pharm.), is an antiviral drug used in Japan to treat influenza. It was developed and is now produced by Toyama Chemical Co (FujiFilm Group) and was approved for medical use in Japan in 2014 [Shiraki K., Daikoku T. Favipiravir, an anti-influenza drug against life-threatening RNA virus infections. Pharmacology & Therapeutics 2020, 107512. doi:10.1016/ j.pharmthera.2020.107512].

In 2016 FujiFilm licensed FVP to the Chinese pharmaceutical company Zhejiang Hisun [E.J. Lane (June 22, 2016). "Fujifilm in Avigan API license with Zhejiang Hisun Pharmaceuticals". Retrieved April 20, 2020.].

In 2019 the FVP became the generic.

In 2010, the PCT international application JP 2010/054191 (12.03.2010) was published [WO 2010/104170 (16.09.2010)], which issued RU 2527766 C2 for "Tablets and granulated powders containing 6-fluoro-3-hydroxy-2-pyrazincarboxamide".

On March 15, 2020, FVP was approved in China for the treatment of coronavirus. [Yangfei Z. "Potential coronavirus drug approved for marketing". Chinadaily.com.cn. Retrieved 2020-03-21].

As of April 30, 2020, the COVID-19 coronavirus affected 210 countries and territories, with a total of 3,308,233 cases of SARS-CoV-2 infected people, of which 1,042,819 people recovered and 234,105 people died [https://www.worldometers.info/coronavirus/?utm_campaign=homeAdvegas1].

Given that SARS-CoV-2 poses a serious threat to the world's public health and economy, it seems appropriate to search for novel potent anti-coronavirus drugs.

The known coated 200-mg tablets Avigan of Toyama Chemical Co., LTV Toyama are protected by the RU 2527766 patent, according to which the content of FVP in the tablet is claimed to be 50-95%. However, in all the 19 examples presented, the content of FVP in tablets varies in the range of 70-80%.

The subject of this invention is an anti-RNA virus, including anti-SARS-CoV-2 virus, pharmaceutical composition Avifavir in tablets or capsules containing less than 50 wt% micronized FVP, with the remainder being excipients.

A preferred form of Avifavir is a coated tablet or a capsule containing less than 45% micronized FVP with a particle size of less than 60 microns, and including 200 mg, 300 mg, 400 mg, or 600 mg FVP of better solubility.

Fillers, disintegrants, binders, glidants, lubricants and coatings can be used as excipients.

More preferable is Avifavir in the form of coated tablets, comprising 43.3% FVP, 42.1% microcrystalline cellulose, 5.8% croscarmellose sodium, 4.9% povidone, 0.7% magnesium stearate, 0.6% silicon dioxide colloidal, and 2.6% film coating.

As can be seen from Table 1, a reduction in FVP particle size in the pharmaceutical composition in the form of a coated tablet leads to a significant improvement in its main parameter-the time of release of the PPV from the tablet in various media. Indeed, the time of FVP release from tablet 3 containing the minimum FVP particle size is significantly greater than the time of FVP release from tablets 1 and 2, in which the particle size exceeds 60 microns.

It should be noted that reduced FVP content also leads to a reduction in the time of FVP release from the pharmaceutical composition in the form of a coated tablet. Thus, the FVP dissolution percentage from coated tablets in a solution with pH 4.5 in examples 2 and 3 (Table 1) of the RU 2527766 patent containing 79% and 86% FVP is 93.5% and 86.7%, respectively, for 15 minutes. At the same time, tablet 3 of this invention containing 43.3% FVP releases a higher percentage of FVP (98.1%, Table 1) and three times faster (in 5 minutes).

A similar pattern is observed when comparing the time of FVP release (95.1%-99.7% for 15 minutes) from coated tablets in a solution with a pH of 4.5 in examples 4 ÷ 10 (Table. 2) from the RU 2527766 patent, each containing 77.5% FPV. At the same time, tablet 3 of this invention containing 43.3% FVP releases 100% FVP in 10 minutes (Table 1).

We note that tablets 3 of this invention release FVP in solutions with pH 1.2 and pH 6.8 just as fast and almost as much (Table 1).

**Table 1. Avifavir coated tablet formulations and FVP dissolution percentage depending on FVP particle size, solution acidity, and the time of mixing of the latter**

| **Composition** | | | **Avifavir coated tablet formulations, mg** | | |
|---|---|---|---|---|---|
| | | | **1*** | **2**** | **3*** |
| FVP | | | 200.00 (42.28%) | 200.00 (45.8%) | 200.00 (43.3%) |
| FVP microcrystal size, µm | | | 67.1 | 211.0 | 40-50 |
| Filler: MCC 102 microcrystalline cellulose | | | 213.14 | - | 194.65 |
| Filller: Prosolv SMCC 90 microcystalline cellulose | | | - | 194.8 | |
| Disintegrant: croscarmellose sodium | | | - | 21 | 27.0 |
| Glidant: Aerosil 200 | | | 2.275 | 1.26 | |
| Disintegrant: L-HPC LH-21 | | | 22.75 | - | |
| Binder: Povidon-K30 | | | 13.65 | - | 22.5 |
| Lubricant: magnesium stearate | | | 3.185 | 2.94 | 3.15 |
| Silicon dioxide colloidal (USP/NF, Ph.Eur.) | | | | | 2.7 |
| Coating: Opadry 85F38183 yellow | | | 18.2 | 16.8 | 12.0 |
| Total | | | 473.20 | 436.80 | 462.0 |

| **Medium***** | **Time** | | **FVP dissolution percentage** | | |
|---|---|---|---|---|---|
| pH 1.2 | 5 min | | 36.6 | 62.1 | 89.6 |
| | 10 min | | 73.8 | 76.0 | 100.5 |
| | 15 min | | 81.6 | 82.7 | 101.6 |
| pH 4.5 | 5 min | | 50.0 | 78.3 | 98.1 |
| | 10 min | | 83.7 | 83.5 | 100.6 |
| | 15 min | | 89.3 | 85.3 | 101.2 |
| pH 6.8 | 5 min | | 45.7 | 70.2 | 100.0 |
| | 10 min | | 83.2 | 75.0 | 101.8 |
| | 15 min | | 90.1 | 77.5 | 102.4 |
| ** micronized FVP and wet granulation, **direct pressing. | | | | | |
| ***The study was carried out on a paddle apparatus at 75 rpm and 37±0.5°C. Dissolution medium pH 1.2-0.2%: sodium chloride solution in 0.1 M hydrochloric acid solution. Dissolution medium pH 4.5: sodium acetate buffer solution (quality control medium). Dissolution medium pH 6.8: phosphate buffer solution. | | | | | |

In addition, due to the reduction of FVP content in the Avifavir pharmaceutical composition, it became possible to significantly improve the technological properties of the granulate (pharmaceutical composition), such as flowability and compressibility, to enable high productivity of the pressing process and high uniformity of the active ingredient in dosage units.

The technology and composition of Avifavir tablets allow one to reduce production time. The high level of productivity of the Tableting and Film Coating stages is enabled by the excellent technological properties of the granulate and the use of a polyvinyl alcohol-based film coating, which permits to apply a more concentrated suspension with a solid content of up to 20%.

This invention is illustrated by, but not limited to, the following examples.

**Example 1.** Preparation of an Avifavir pharmaceutical composition in capsules containing 200 mg (45%) of FVP. Micronized FVP with a microcrystal size of 40-50 µm (200 g) and lactose powder (250 g) are carefully mixed. The resulting powder mixture is packed in 450 mg gelatin capsules of suitable size, each containing 200 mg (44.4%) of FVP.

**Example 2.** Preparation of an Avifavir pharmaceutical composition in coated tablets containing 200 mg, 300 mg, 400 mg, or 600 mg of FVP (formulation 3 in Table 1).

All starting materials are weighed, and magnesium stearate for dusting is sieved. Micronized FVP with a microcrystal size of 40-50 µm (200 g), MCC 102 microcrystalline cellulose (194.65 g), croscarmellose sodium (27.0 g), and 2.7 g of colloidal silicon dioxide (USP/NF, Ph.Eur.) are sequentially loaded in a granulating mixer, and the components are stirred until a homogeneous mixture is obtained. A previously prepared 6% solution of povidone K30 (22.5 g) is added to the granulator mixer in full at constant stirring, until the final granulation point is reached. Wet granulate is calibrated through a 2.0 mm sieve. The calibrated wet granulate is dried to the specified residual humidity. The dried granulate is calibrated through a 0.5 mm sieve to set the optimal fractional composition. The resulting granulate is powdered in a mixer with pre-sifted 3.15 g of magnesium stearate. The powdered mixture is divided into three parts and tableted on a rotary tablet press. The resulting core tablets with a mass of ≈450 mg, ≈675 mg, and ≈1350 mg, containing 200 mg, 300 mg, 400 mg, or 600 mg FVP, respectively, each with a hardness of 60 N, an abrasion of max 5% and a disintegration of max 3 min are passed to the coating stage. The film coat (Opadry 85F38183 yellow) is applied in a coater to attain the specified weight of the Avifavir coated tablet weighing 462 mg, 693 mg, or 1386 mg, respectively.

Avifavir pharmaceutical compositions in coated tablets as per formulations 1 and 2 are obtained similarly (Table 1).

**Example 3.** Kinetics of dissolution of Avifavir coated tablets containing 200 mg FVP in three buffer media.

The study of the kinetics of dissolution of Avipiravir in coated tablets containing 200 mg FVP was carried out in accordance with the "Guidelines for the Examination of Medicinal Products [Volume 1. Moscow: Grif and K., 2013, 328 pp., Chapter 7. 5. Guide to the examination of medicines. Volume 3. Moscow: POLYGRAPHPLUS Publ., 2014, 344 pp., Chapter 11.] in three buffer media with pH values of 1.2, 4.5 and 6.8 modeling the main areas of the gastrointestinal tract, in which the release and absorption of the active ingredient occurs. The following media were used: 0.2 % sodium chloride solution in 0.1 M hydrochloric acid solution with pH 1.2; sodium acetate buffer solution with pH 4.5 (quality control medium); and phosphate buffer solution with pH 6.8; all the solutions were prepared in accordance with the requirements of EP. 7. 0. 5.17.1 "Recommendations on dissolution testings." Sampling time points were selected in such a way as to provide a reliable description of the dissolution profile with a gradual increase and subsequent reaching the level of full release (at least 85% of the active ingredient) or a plateau. In studying dissolution kinetics, the following time points were selected: 5 min, 10 min, 15 min, 20 min, and 30 min. To obtain statistically reliable results for each drug, the test was performed on 12 dosage form units. The quantitative content of FVP released into the solution medium was determined by HPLC. The calculations took into account the change in the volume of the dissolution medium.

The study of the dissolution kinetics was performed on a DT 828 Dissolution Tester (Erweka, Germany) intended to evaluate the dissolution of tablets / capsules. Quantitative determination was performed using liquid chromatographs: Agilent 1260 (Agilent Technologies, USA) using OpenLab ChemStation software and LC-20A Prominence (Shimadzu, Japan) using LabSolutions software. Auxiliary equipment included: laboratory scales MV210A (SartoGosm, Russia), Acculab VIC-210d2 (Acculab, Sartorius Group, USA), and Quintix 64-1ORU (Sartorius Group, Germany); SEVEN MULTI pH meter (Mettler Toledo, Switzerland). Statistical processing of the experiment results was performed using the Microsoft Office Excel 2007 package. We used measuring laboratory utensils of classes "A" (measuring flasks with a capacity of 50, 500, 1000 ml), "AS" (analytical pipettes 1 and 5 ml) and "B" (measuring cylinders 100, 250 and 1000 ml).

The test was performed in accordance with the requirements of State Pharmacopoeia XIV, GPM 1.4.2.0014.15 "Dissolution for solid dosage forms" [SPh XIV. Volume 2, 2018. - 2164 p., GPM.1.4.2.0014.15 Dissolution for solid dosage forms.], "Guide to the examination of medicines" [Guide to the examination of medicines. Volume 1. Moscow: Grif and K., 2013. - 328 p., Chapter 7. 5. Guide to the examination of medicines. Volume 3. Moscow: POLIGRAFPLUS Publ., 2014, 344 pp., Chapter 11.], as well as in accordance with the recommendations of the scientific and practical guide for the pharmaceutical industry "Dissolution test in the development and registration of medicines" edited by I. E. Shokhin ["Dissolution Test" in the Development and Registration of Medicines. Scientific and Practical Guide for the Pharmaceutical Industry / Ed. I.E. Shokhin. Moscow, Pero Publ., 2015, 320 pp].

The test conditions were as follows. Instrument type: paddle apparatus; temperature: 37±0.5°C; medium volume: 900 ml; rotation speed: 75 rpm; sampling time points: 5, 10, 15, 20, and 30 min. The results obtained from the study of FVP release from three Avipiravir tablet formulations depending on the size of the FVP particle size, the acidity of the solutions and the mixing time are presented in Table 1.

## Claims

1. An anti-RNA virus, including anti-SARS-CoV-2 virus, pharmaceutical composition Avifavir in tablets or capsules containing less than 50 wt % of micronized favipiravir, with the remainder being excipients.

2. Avifavir according to Claim 1 in tablets or capsules containing less than 40-45% of micronized favipiravir with a particle size less than 60 µm.

3. Avifavir according to Claims 1, 2 containing fillers, disintegrants, binders, glidants, lubricants and coatings as excipients.

4. Avifavir according to Claims 1 ÷ 3 in coated tablets containing 43÷44% favipiravir, 5.5÷6.0% croscarmellose sodium, 4.8÷5.0% povidone, 0.6÷0.8% magnesium stearate, 0.5÷0.7% silicon dioxide colloidal, 2.5÷2.7% film coating, the remainder being microcrystalline cellulose, containing 200 mg, 300 mg, 400 mg, or 600 mg of FVP.
